# EUROPÄISCHE PATENTSCHRIFT

(11) **EP 2 358 664 B1**
(45) Veröffentlichungstag und Bekanntmachung des Hinweises auf die Patenterteilung: **25.03.2020**
(21) Anmeldenummer: 09756729.1
(22) Anmeldetag: 19.11.2009
(51) Int. Cl.: C08F 220/56, C07C 231/02, C07C 233/09

(54) **VERFAHREN ZUR HERSTELLUNG VON N-ISOPROPYL(METH)ACRYLAMID**
METHOD FOR PRODUCING N-ISOPROPYL(METH)ACRYLAMIDE
PROCÉDÉ DE PRODUCTION DE MÉTHACRYLAMIDE N-ISOPROPYLIQUE

(30) Priorität: 15.12.2008 DE 102008054612
(43) Veröffentlichungstag der Anmeldung: 24.08.2011
(60) Teilanmeldung: 20156880.5
(73) Patentinhaber: Evonik Operations GmbH, 45128 Essen (DE)
(72) Erfinder: KNEBEL, Joachim, 64665 Alsbach-Hähnlein (DE); KARNBROCK, Wilhelm, 64625 Bensheim (DE); KERSCHER, Volker, 64354 Reinheim (DE)
(74) Vertreter: Evonik Patent Association
(86) Internationale Anmeldenummer: PCT/EP2009/065435
(87) Internationale Veröffentlichungsnummer: WO 2010/072480

(56) Entgegenhaltungen:
- DE-A1-102006 047 617
- US-A1- 2004 024 152
- WEN-HAI HONG ET.AL.: "Identification of aliphatic amines from ratse of cinnamoylation" J. PHARM. SCI., Bd. 57, Nr. 10, 1968, Seiten 1789-1790, XP002581558

## Beschreibung

### Gebiet der Erfindung

Es wird ein Verfahren beschrieben, das es erlaubt, N-Isopropyl(meth)acrylamid auf einfache Weise in hoher Reinheit und Ausbeute zu gewinnen, indem man Methacrylsäureanhydrid mit Isopropylamin zur Reaktion bringt, in einem Lösungsmittel. Das Amid fällt während der Reaktion aus und kann durch Filtration isoliert werden. Ein weiterer Produktanteil kann durch Neutralisation der als Nebenprodukt anfallenden Methacrylsäure aus dem Filtrat ausgefällt werden und so zusätzlich gewonnen werden. Alternativ kann die Methacrylsäure destillativ von darin gelöstem Produkt getrennt werden.

Überraschenderweise bildet sich im Gegensatz zum Lehrbuchwissen bei der äquimolaren Umsetzung der Reaktanden nahezu kein N-Isopropylammoniummethacrylat.

### Stand der Technik

Die **Ritter-Reaktion** dient zur Herstellung von Amiden aus Nitrilen und Substraten, die Carbeniumionen bilden können (wie zum Beispiel tertiäre oder sekundäre Alkohole in Gegenwart von starken Mineralsäuren). So kann aus (Meth)acrylnitril und Isopropanol N-Isopropyl(meth)acrylamid hergestellt werden. Die DE 31 31 096 beschreibt dieses Verfahren. Zur Aufarbeitung muss die als Lösungsmittel fungierende Säure neutralisiert werden, was große Mengen Abfallsalz erzeugt. Obendrein enthält das Produkt Verunreinigungen, zum Beispiel Methacrylamid.

Wen-Hai Hong et.al.: "Identification of aliphatic amines from ratse of cinnamoylation", J. Pharm.SCI. Bd. 57, Nr. 10, 1968 beschreibt die Umsetzung von trans- Zimtsäureanhydrid mit verschiedenen Aminen in Acetonitril. Dabei verläuft die Umsetzung einiger Amine in Acetonitril sehr schnell. Zur besseren Kontrolle der Reaktion muss die Konzentration der Reaktanten herabgesetzt werden. Für einen großtechnischen Prozess ist diese Maßnahme kontraproduktiv. Zudem wird das Anhydrid mehrfach in Benzol umkristallisiert, um es wasserfrei zu bekommen. Das Lösungsmittel Acetonitril wird über Phosphorpentoxid destilliert, um das Restwasser zu entfernen. Die Amine werden mittels Destillation aufgereinigt. Alle genannten Maßnahmen führen dazu, dass wasserfreie Reaktionsbedingungen vorliegen.

DE 102006047617 beschreibt die Herstellung von basischen Amiden oder Imiden durch Umsetzung von Aminen mit ethylenisch ungesättigten Carbonsäuren zu Ammoniumsalzen, welche durch Mikrowellenbestrahlung zu Amiden bzw. Imiden umgesetzt werden. Als Eduktamine werden Amine mit zwei oder mehr Aminogruppen verwendet. Von diesen Aminogruppen ist mindestens eine tertiär. Mindestens eine Aminogruppe trägt ein oder zwei H-Atome.

Die US 4859796 beschreibt ein Verfahren zur Synthese von N-Dialkylaminoalkyl(meth)acrylamiden, dadurch gekennzeichnet, dass (Meth)acrylsäureanhydrid in Gegenwart von Polymerisationsinhibitoren mit Diaminen bei Temperaturen zwischen 70 und 100°C und in Gegenwart von Sauerstoff umgesetzt wird.

Die Umsetzung mit thermisch stabileren Diaminen zu ebenfalls stabilen Dialkylamiden ist nicht übertragbar auf die Herstellung von Amiden der (Meth)acrylsäure durch Umsetzung von primären oder sekundären Aminen mit Anhydriden der (Meth)acrylsäure.

### Aufgabenstellung

### Durchführung der Erfindung

Man erhält das substituierte (Meth)acrylamid auf einfache Weise, indem man das Anhydrid, in Wasser vorlegt und das Amin, unter Kühlung, zudosiert.

Nach Ende der Zugabe ist in der Regel bereits ein Teil des entstandenen Produkts auskristallisiert und kann, nach Kühlen der Produktlösung zur Vervollständigung der Fällung, abfiltriert werden. Zur Erhöhung der Ausbeute kann die in der Mutterlauge enthaltene (Meth)acrylsäure neutralisiert werden, was zu einer weiteren Ausfällung von Produkt führt. Die Produktbildung erfolgt nahezu quantitativ. Das erhaltene substituierte (Meth)acrylamid besitzt eine hohe Reinheit, die gewöhnlich oberhalb von 95 % liegt. Wegen der Polymerisationsempfindlichkeit der Monomeren ist es zweckmäßig, dabei die thermische Belastung zu minimieren. Reinheit und Ausbeute der erhaltenen Monomere sind höher als bei Herstellung nach bekannten Verfahren wie der Ritter-Reaktion oder dem Einsatz von Carbonsäurechlorid als Rohstoff.

### Die Ausgangsstoffe

Als Anhydrid kommen Acrylsäureanhydrid, Methacrylsäureanhydrid in Frage.

Das Methacrylsäureanhydrid wird z. B. von der Evonik Röhm GmbH in den Handel gebracht.

### Die Amine

Als Amine können primäre Amine und sekundäre Amine eingesetzt werden. Als primäre Amine kommen primäre ggf. substituierte aliphatische Amine in Frage, wie beispielsweise Methylamin, Ethylamin, Propylamin, Butylamin, Pentylamin, Hexylamin, Heptylamin, Octylamin, Dodecylamin, Isopropylamin, Isobutylamin und Benzylamin sowie Allylamin.

Ferner können primäre cycloaliphatische Amine eingesetzt werden, wie beispielsweise Cyclopropylamin, Cyclobutylamin, Cyclopentylamin und Cyclohexylamin.

Als primäre aromatische Amine können Anilin, die isomeren Aminotoluole, einzeln oder in Mischungen und die isomeren Xylidine, einzeln oder in Mischungen eingesetzt werden. Diese Verbindungen können gegebenenfalls durch ein oder mehrere Halogene substituiert sein.

Sekundäre aliphatische Amine, wie beispielsweise Dimethylamin, Methylethylamin, Diethylamin, Dipropylamin, Diisopropylamin, Dibutylamin, Dipentylamin, Dihexylamin, Diheptylamin und Dioctylamin können ebenfalls eingesetzt werden.

### Die Polymerisationsinhibitoren

Polymerisationsinhibitoren sind bereits bekannt. So können beispielsweise 1,4-Dihydroxybenzole zur Stabilisierung zugegeben werden. Es können jedoch auch anders substituierte Dihydroxybenzole zum Einsatz kommen. Allgemein lassen sich derartige Inhibitoren mit der allgemeinen Formel (I) wiedergeben 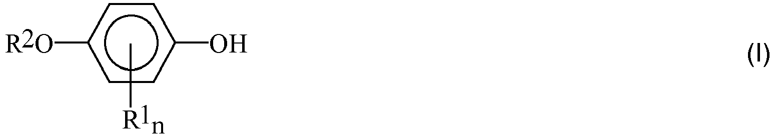 worin
R¹ Wasserstoff, einen linearen oder verzweigten Alkylrest mit eins bis acht Kohlenstoffatomen, Halogen oder Aryl bedeutet, vorzugsweise einen Alkylrest mit eins bis vier Kohlenstoffatomen, besonders bevorzugt Methyl, Ethyl, n-Propyl, iso-Propyl, n-Butyl, iso-Butyl, sec-Butyl, tert.-Butyl, Cl, F oder Br;
n eine ganze Zahl im Bereich von eins bis vier, vorzugsweise eins oder zwei ist; und
R² Wasserstoff, einen linearen oder verzweigten Alkylrest mit eins bis acht Kohlenstoffatomen oder Aryl bedeutet, vorzugsweise einen Alkylrest mit eins bis vier Kohlenstoffatomen, besonders bevorzugt Methyl, Ethyl, n-Propyl, iso-Propyl, n-Butyl, iso-Butyl, sec-Butyl oder tert.-Butyl.

Es können jedoch auch Verbindungen mit 1,4-Benzochinon als Stammverbindung eingesetzt werden. Diese lassen sich mit der Formel (II) beschreiben 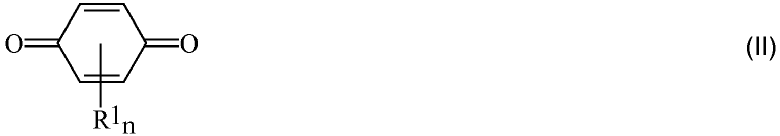 worin
R¹ einen linearen oder verzweigten Alkylrest mit eins bis acht Kohlenstoffatomen, Halogen oder Aryl bedeutet, vorzugsweise einen Alkylrest mit eins bis vier Kohlenstoffatomen, besonders bevorzugt Methyl, Ethyl, n-Propyl, iso-Propyl, n-Butyl, iso-Butyl, sec-Butyl, tert.-Butyl, Cl, F oder Br; und
n eine ganze Zahl im Bereich von eins bis vier, vorzugsweise eins oder zwei ist. Ebenso werden Phenole der allgemeinen Struktur (III) eingesetzt. 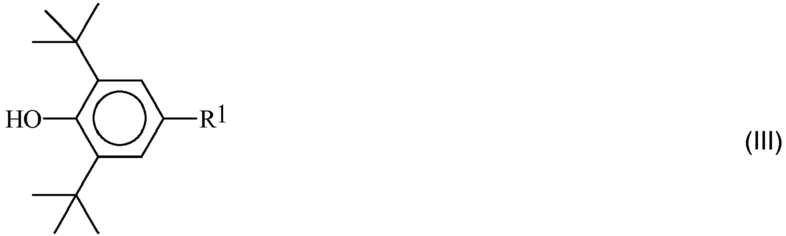
worin
R¹ einen linearen oder verzweigten Alkylrest mit eins bis acht Kohlenstoffatomen, Aryl oder Aralkyl, Proprionsäureester mit 1 bis 4 wertigen Alkoholen, welche auch Heteroatome wie S, O und N enthalten können, vorzugsweise einen Alkylrest mit eins bis vier Kohlenstoffatomen, besonders bevorzugt Methyl, Ethyl, n-Propyl, iso-Propyl, n-Butyl, iso-Butyl, sec-Butyl, tert.-Butyl, bedeutet.

Eine weitere vorteilhafte Substanzklasse stellen sterisch gehinderte Phenole auf Basis von Triazinderivaten der Formel (IV) dar: 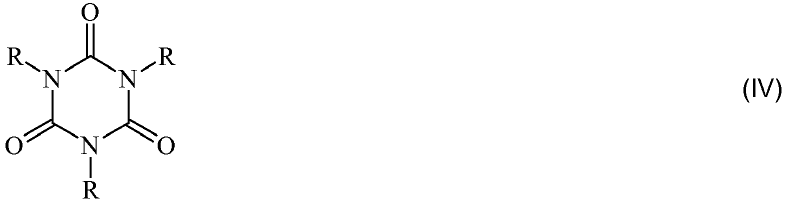 mit R = Verbindung der Formel (V) 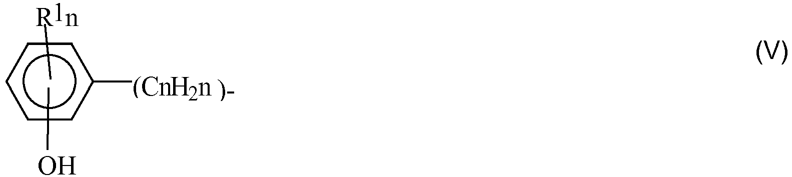 worin
R¹ = CₙH₂ₙ₊₁
mit n = 1 oder 2 ist.
Eine weitere Gruppe von bekannten Inhibitoren sind Amine, insbesondere sterisch gehinderte Amine.

Zu diesen gehören insbesondere Phenylendiamine, die durch Formel (VI) darstellbar sind 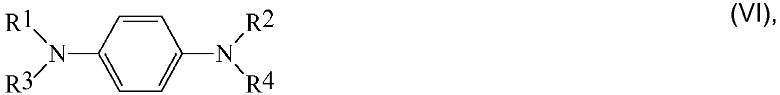 worin R¹, R², R³ und R⁴ unabhängig Wasserstoff sowie Alkyl-, Aryl-, Alkaryl-, Aralkyl-Reste mit jeweils bis zu 40, vorzugsweise bis zu 20 Kohlenstoffatomen darstellen, wobei vorzugsweise mindestens einer der Reste R¹, R², R³ und R⁴ Wasserstoff ist.

Beispielhafte p-Phenylendiamine umfassen p-Phenylendiamin worin die Reste R¹, R², R³ und R⁴ Wasserstoff sind; N-Phenyl-N'-alkyl-p-phenylendiamine wie beispielsweise, N-Phenyl-N'-methyl-p-phenylendiamin, N-Phenyl-N'-ethyl-p-phenylendiamin, N-Phenyl-N'-propyl-p-phenylendiamin, N-Phenyl-N'-isopropyl-p-phenylendiamin, N-Phenyl-N'-n-butyl-p-phenylendiamine, N-Phenyl-N'-isobutyl-p-phenylendiamin, N-Phenyl-N'-sec-butyl-p-phenylendiamin, N-Phenyl-N'-tert-butyl-p-phenylendiamin, N-Phenyl-N'-n-pentyl-p-phenylendiamin, N-Phenyl-N'-n-hexyl-p-phenylendiamin, N-Phenyl-N'-(1-methylhexyl)-p-phenylendiamin, N-Phenyl-N'-(1,3-dimethylbutyl)-p-phenylendiamin, N-Phenyl-N'-(1,4-dimethyl-pentyl)-p-phenylendiamin; N-Phenyl-N',N'-dialkyl-p-phenylendiamine, wie beispielsweise N-Phenyl-N',N'-dimethyl-p-phenylendiamin, N-Phenyl-N',N'-diethyl-p-phenylendiamin, N-Phenyl-N',N'-di-n-butyl-p-phenylendiamin N-Phenyl-N',N'-di-sec-butyl-p-phenylendiamin, N-Phenyl-N'-methyl-N'-ethyl-p-phenylendiamin; N,N-Dialkyl-p-phenylendiamine, wie beispielsweise N,N-Dimethyl-p-phenylendiamin und N,N'-Diethyl-p-phenylendiamin; N,N'-Dialkyl-p-phenylendiamine, wie beispielsweise N,N'-Diisopropyl-p-phenylendiamin, N,N'-Diisobutyl-p-phenylendiamin; N,N'-Diaryl-phenylendiamine, wie beispielsweise N,N'-Diphenyl-p-phenylendiamin; N,N,N'-Trialkyl-p-phenylendiamine, wie beispielsweise N,N,N'-Trimethyl-p-phenylendiamin, N,N,N'-Triethyl-p-phenylendiamin.

Darüber hinaus bilden Phenazin-Farbstoffe eine weitere bevorzugte Gruppe. Diese umfassen insbesondere Indulin und Nigrosin. Nigrosin entsteht durch Erhitzen von Nitrobenzol, Anilin und salzsaurem Anilin mit metallischem Eisen und FeCl₃. Hierbei sind alkohollösliche Anilinfarbstoffe bevorzugt, die beispielsweise 5 Benzolkerne umfassen können, wie Dianilido-N,N-diphenylphenosafranin. Diese Stoffe sind weithin bekannt und können kommerziell erhalten werden.

Besonders erfolgreich werden die Verbindungen 1,4-Dihydroxybenzol, 4-Methoxyphenol, 2,5-Dichloro-3,6-dihydroxy-1,4-benzochinon, 1,3,5-Trimethyl-2,4,6-tris-(3,5-di-tert.butyl-4-hydroxybenzyl)benzol, 2,6-Di-tert. butyl-4-methylphenol, 2,4-Dimethyl-6-tert. butylphenol, 2,2-Bis [3,5-Bis(1,1-dimethylethyl)-4-hydroxyphenyl-1-oxopropoxymethyl)]1,3-propandiylester, 2,2'-Thiodiethylbis-[3-(3,5-di-tert.butyl-4-hydroxyphenyl)]propionat, Octadecyl-3-(3,5-di-tert.butyl-4-hydroxyphenyl)propionat, 3,5-Bis(1,1-dimethylethyl-2,2-Methylenbis-(4-methyl-6-tert.butyl)phenol, Tris-(4-tert.butyl-3-hydroxy-2,6-dimethylbenzyl)-s-triazin-2,4,6-(1H,3H,5H)trion, Tris (3,5-ditert.butyl-4-hydroxy)-s-triazin-2,4,6-(1H,3H,5H) trion, tert.-Butyl-3,5-dihydroxybenzol oder Diphenyl-p-phenylendiamin (DPPD) sowie 4-Hydroxy-2,2,6,6-tetramethylpiperidin-1-oxyl eingesetzt, wobei hiervon wiederum ganz besonders zweckmäßig Hydrochinonmonomethylether (4-Methoxyphenol) ist.

Die genannten Inhibitoren sind kommerziell erhältlich.

Als Grundstabilisierung für ethylenisch ungesättigte Verbindungen können die genannten Verbindungen allein oder in Mischung von zwei oder mehr Verbindungen eingesetzt werden. Sofern es sich um phenolische Verbindungen handelt, ist die Anwesenheit von Sauerstoff im Reaktionsgemisch erforderlich, um eine ausreichende Wirksamkeit gegen Polymerisation sicherzustellen. Dabei ist die Verwendung von Luft als Sauerstoffquelle besonders bevorzugt.

### Das Lösungsmittel

Als Lösungsmittel wird Wasser verwendet.

Wasser kommt als inertes Lösungsmittel in Betracht, da es unter den Reaktionsbedingungen der Amidherstellung nur wenig Hydrolyse des (Meth)acrylsäureanhydrids bewirkt.

### Beispiele

### Vergleichsbeispiel 1: Herstellung von N-Isopropylmethacrylamid

In einem 2l-Fünfhalsrundkolben mit mechanischem Rührer, Tropftrichter, Lufteinleitrohr, Innentemperaturfühler und Rückflußkühler legt man 463 g (3 mol) Methacrylsäureanhydrid, 504 g (6 mol) Cyclohexan sowie als Inhibitor 0,077 g (120 ppm bez. auf Reaktanden) 2,6-Di(tert.butyl)-4-methylphenol vor und dosiert in 1,25 h bei einer Temperatur unter 20°C 177 g (3 mol) Isopropylamin unter Rühren und Einleiten eines langsamen Luftstroms zu. Dabei scheiden sich Kristalle ab. Nach Ende der Zugabe kühlt man die Suspension auf 4°C bis 8°C ab und rührt noch 2,5 h zur Vervollständigung der Fällung. Danach wird filtriert und die Kristalle werden zweimal mit je 60 g Cyclohexan gewaschen und an der Luft getrocknet. Ausbeute 188 g (49%) N-Isopropylmethacrylamid, Reinheit 97,8 % (durch Gaschromatographie ermittelt).

Die Mutterlauge wird zur Neutralisation der enthaltenen Methacrylsäure mit 280 g 30%iger Natronlauge versetzt, wobei sich zwei Phasen bilden. Man rührt 0,5 h bei Raumtemperatur, wobei kristalliner Feststoff ausfällt. Die Suspension wird zur Vervollständigung der Fällung 12 h bei 5°C gelagert. Danach wird filtriert und der Filterkuchen wird an der Luft getrocknet.

Ausbeute: 165 g (43% d. Th.) N-Isopropylmethacrylamid, Reinheit 93,8 % (durch Gaschromatographie ermittelt).

### Vergleichsbeispiel 2: Herstellung von N-Dodecylmethacrylamid

In einem 11-Fünfhalsrundkolben mit mechanischem Rührer, Tropftrichter, Lufteinleitrohr, Innentemperaturfühler und Rückflußkühler legt man 300 g (1,95 mol) Methacrylsäureanhydrid sowie als Inhibitor 0,06 g (120 ppm bez. auf Produkt) 2,6-Di(tert.butyl)-4-methylphenol sowie 0,03 g (60 ppm bez. auf Produkt) Hydrochinonmonomethylether sowie 0,003 g (6 ppm bez. auf Produkt) 4-Hydroxy-2,2,6,6-tetramethylpiperidin-1-oxyl vor und dosiert in 1,3 h bei einer Temperatur von 30...35°C 361 g (1,95 mol) aufgeschmolzenes (Fp. 25..28°C) N-Dodecylamin unter Rühren und Einleiten eines langsamen Luftstroms zu. Nach Ende der Zugabe rührt man den flüssigen leicht viskosen Ansatz 3 h bei 40..45°C nach und kühlt auf Raumtemperatur ab. Zum Abdestillieren der Methacrylsäure versieht man das Reaktionsgefäß mit einer Destillationsbrücke und erhitzt im Ölpumpenvakuum (1 mbar) innerhalb von 3h auf 97°C Sumpftemperatur, wobei 154 g Methacrylsäure (92 % d. Th.) erhalten werden. Der Destillationsrückstand besteht aus 490 g N-Dodecylmethacrylamid (99 % d. Th.) mit einer gaschromatographisch bestimmten Reinheit von 98%.

### Vergleichsbeispiel 3: Herstellung von N-Isopropylmethacrylamid

In einem 11-Vierhalsrundkolben mit mechanischem Rührer, Tropftrichter, Innentemperaturfühler und Rückflußkühler legt man 300 g (1,95 mol) Methacrylsäureanhydrid sowie als Inhibitor 0,06 g (120 ppm bez. auf Produkt) 2,6-Di(tert.butyl)-4-methylphenol sowie 0,03 g (60 ppm bez. auf Produkt) Hydrochinonmonomethylether sowie 0,003 g (6 ppm bez. auf Produkt) 4-Hydroxy-2,2,6,6-tetramethylpiperidin-1-oxyl vor und dosiert in 3 h bei einer Temperatur von max. 30°C 115 g (1,95 mol) Isopropylamin unter Rühren zu. Nach Ende der Zugabe rührt man den flüssigen leicht viskosen Ansatz 3 h bei 40°C nach und kühlt auf Raumtemperatur ab. Die ausgefallenen Kristalle werden abgesaugt und mit 157 g Cyclohexan gewaschen, das mit der Mutterlauge vereinigt wird. Man erhält als erste Fraktion 114 g (46% d. Th.) N-Isopropylmethacrylamid mit einem Schmelzpunkt von 89 °C und einer gaschromatographisch bestimmten Reinheit von 99 %. Zur Aufarbeitung der Mutterlauge führt man diese einem Labordünnschichtverdampfer DS 25 (Fa. NGW, Wertheim) mit aufgesetzter Vigreuxkolonne (Länge 10 cm) zu und zieht bei einer Heizmanteltemperatur von 130°C und einem Druck von 1 mbar das Cyclohexan und die Methacrylsäure ab. Der Destillationsrückstand erstarrt beim Abkühlen auf RT und besteht aus 130 g N-Isopropylmethacrylamid (52 % d. Th.) mit einer gaschromatographisch bestimmten Reinheit von 97 %.

### Vergleichsbeispiel 4: Herstellung von N-Isopropylmethacrylamid aus Methacrylsäurechlorid

In einem 11-Vierhalsrundkolben mit mechanischem Rührer, Tropftrichter, Innentemperaturfühler und Rückflußkühler legt man 500 g (1mol) 2n Natronlauge, 59 g (1 mol) Isopropylamin sowie als Inhibitor 0,1 g 2,4-Dimethyl-6-(tert.butyl)phenol vor und dosiert bei Raumtemperatur 104 g (1 mol) Methacrylsäurechlorid unter Rühren zu. Nach Ende der Zugabe rührt man den Ansatz 1 h nach, fügt nochmals 10 g Natriumhydroxid hinzu und rührt weitere 30 min. Die ausgefallenen Kristalle werden abgesaugt und an der Luft getrocknet. Man erhält 108 g (85 % d. Th.) N-Isopropylmethacrylamid mit einer gaschromatographisch bestimmten Reinheit von 90,7%. Als Nebenprodukt sind 9 % eines Additionsprodukts von Methacrylsäurechlorid an N-Isopropylmethacrylamid enthalten.

### Vergleichsbeispiel 5: Herstellung von N-Isopropylmethacrylamid aus Methacrylsäurechlorid

In einem 11-Vierhalsrundkolben mit mechanischem Rührer, Tropftrichter, Lufteinleitrohr, Innentemperaturfühler und Rückflußkühler legt man 300 ml Toluol sowie 118 g (2mol) Isopropylamin und als Inhibitor 0,1 g 2,6-Di(tert.butyl)-4-methylphenol vor und dosiert bei max. 30°C 104 g (1 mol) Methacrylsäurechlorid unter Rühren und Lufteinleitung zu. Das ausgefallene Produkt wird abgesaugt und getrocknet. Man erhält 65 g (51% d. Th.) N-Isopropylmethacrylamid mit einer gaschromatographisch bestimmten Reinheit von 82,4%. Als Nebenprodukt sind 1,3 % eines Additionsprodukts von Methacrylsäurechlorid an N-Isopropylmethacrylamid sowie 11 % Methacrylsäureanhydrid enthalten.

### Vergleichsbeispiel 6: Herstellung von N-Isopropylmethacrylamid aus Methacrylnitril nach Ritter in 100%iger Schwefelsäure

In einem 2l-Vierhalsrundkolben mit mechanischem Rührer, Tropftrichter, Lufteinleitrohr, Innentemperaturfühler und Rückflußkühler legt man 515 g (5,25 mol) 100%ige Schwefelsäure vor und dosiert innerhalb von 2,5 h ein Gemisch von 168 g (2,5 mol) Methacrylnitril, 180 g (3 mol) Isopropanol und 0,18 g 2,6-Di(tert.butyl)-4-methylphenol unter Rühren und Lufteinleiten zu. Die exotherme Reaktion wird durch Kühlen im Bereich von 22 bis 25°C gehalten. Nach Ende der Zugabe lässt man 1h bei 30°C nachreagieren, erwärmt dann auf 60°C und hält das Reaktionsgemisch 1 h bei dieser Temperatur. Danach kühlt man auf Raumtemperatur und gibt innerhalb von 70 min. 750 g Wasser unter Kühlen hinzu, so dass die Temperatur nicht ansteigt. Man überführt den Ansatz in einen 4l-Kolben und gibt weitere 925 g Wasser zu, wobei sich Feststoff abscheidet. Mit 50%iger Natriumhydroxidlösung (ca. 815 g) wird das Gemisch innerhalb von 120 min bei max. 30°C neutralisiert (pH=7). Man kühlt auf 18 °C, dann wird das ausgefallene Produkt abgesaugt und viermal mit je 320 ml kaltem Wasser gewaschen. Der erhaltene Kristallbrei wird abgepresst. Man erhält 1136 g Produkt, das neben N-Isopropylmethacrylamid 42 % Wasser und 22 % Natriumsulfat enthält. Der N-Isopropylmethacrylamidanteil weist eine gaschromatographisch bestimmte Reinheit von 93,5% auf und enthält 5,9 % Methacrylamid.

Verbindungen können als Monomere zur Herstellung von Polymerisaten und Copolymerisaten verwendet werden. Ferner können die Monomere zu Polymeren polymerisiert oder in Mischungen copolymerisiert werden, die als Gashydratbildungsinhibitoten verwendet werden.

## Patentansprüche

1. Verfahren zur Herstellung von Amiden der (Meth)acrylsäure,
**dadurch gekennzeichnet, dass**
man das Anhydrid der (Meth)acrylsäure mit einem primären oder sekundären Amin unter Kühlung in Wasser umsetzt und das entstehende Amid mittels Filtration abtrennt.

2. Verfahren zur Herstellung von Amiden der (Meth)acrylsäure gemäß Anspruch 1,
**dadurch gekennzeichnet, dass**
man das Anhydrid der (Meth)acrylsäure mit einem primären oder sekundären Amin ausgewählt aus der Gruppe der aliphatischen Amine, wie Methylamin, Ethylamin, Propylamin, Butylamin, Pentylamin, Hexylamin, Heptylamin, Octylamin, Dodecylamin, Isopropylamin, Isobutylamin und Benzylamin sowie Allylamin, primären cycloaliphatischen Amine, wie Cyclopropylamin, Cyclobutylamin, Cyclopentylamin und Cyclohexylamin, aromatischen Amine wie Anilin, die isomeren Aminotoluole, einzeln oder in Mischungen und die isomeren Xylidine, einzeln oder in Mischungen, die gegebenenfalls durch ein oder mehrere Halogene substituiert sein können und der sekundären aliphatischen Amine, wie Dimethylamin, Methylethylamin, Diethylamin, Dipropylamin, Diisopropylamin, Dibutylamin, Dipentylamin, Dihexylamin, Diheptylamin und Dioctylamin umsetzt.

3. Verfahren zur Herstellung von Amiden der (Meth)acrylsäure gemäß der Ansprüche 1-2,
**dadurch gekennzeichnet, dass**
man die Mutterlauge aus dem Amidabtrennschritt neutralisiert, um weiteres Amid auszufällen und das entstehende Amid mittels Filtration abtrennt.

4. Verfahren zur Herstellung von Amiden der (Meth)acrylsäure gemäß der Ansprüche 1-2,
**dadurch gekennzeichnet, dass**
man die Mutterlauge aus dem Amidabtrennschritt destillativ von (Meth)acrylsäure befreit und das zurückbleibende Amid mittels Filtration abtrennt.

5. Verfahren zur Herstellung von N-Isopropylmethacrylamid,
**dadurch gekennzeichnet, dass**
man Methacrylsäureanhydrid mit Isopropylamin in Wasser umsetzt und das N-Isopropylmethacrylamid isoliert.

## Claims

1. Process for preparing amides of (meth)acrylic acid,
**characterized in that**
the anhydride of (meth)acrylic acid is reacted, with cooling, in water with a primary or secondary amine, and the amide formed is separated off by means of filtration.

2. Process for preparing amides of (meth)acrylic acid according to Claim 1,
**characterized in that**
the anhydride of (meth)acrylic acid is reacted with a primary or secondary amine selected from the group of the aliphatic amines, such as methylamine, ethylamine, propylamine, butylamine, pentylamine, hexylamine, heptylamine, octylamine, dodecylamine, isopropylamine, isobutylamine and benzylamine and also allylamine, primary cycloaliphatic amines, such as cyclopropylamine, cyclobutylamine, cyclopentylamine and cyclohexylamine, aromatic amines aniline, the isomeric aminotoluenes, individually or in mixtures, and the isomeric xylidines, individually or in mixtures, which may optionally be substituted by one or more halogens, and the secondary aliphatic amines, such as dimethylamine, methylethylamine, diethylamine, dipropylamine, diisopropylamine, dibutylamine, dipentylamine, dihexylamine, diheptylamine and dioctylamine.

3. Process for preparing amides of (meth)acrylic acid according to Claims 1-2,
**characterized in that**
the mother liquor from the amide separation step is neutralized in order to precipitate further amide, and the resulting amide is separated off by means of filtration.

4. Process for preparing amides of (meth)acrylic acid according to Claims 1-2,
**characterized in that**
the mother liquor from the amide separation step is cleared of (meth)acrylic acid by distillation and the amide remaining behind is separated off by means of filtration.

5. Process for preparing N-isopropylmethacrylamide,
**characterized in that**
methacrylic anhydride is reacted in water with isopropylamine and the N-isopropylmethacrylamide is isolated.

## Revendications

1. Procédé de fabrication d'amides de l'acide (méth)acrylique, **caractérisé en ce que** l'anhydride de l'acide (méth)acrylique est mis en réaction avec une amine primaire ou secondaire avec refroidissement dans de l'eau, et l'amide formé est séparé par filtration.

2. Procédé de fabrication d'amides de l'acide (méth)acrylique selon la revendication 1, **caractérisé en ce que** l'anhydride de l'acide (méth)acrylique est mis en réaction avec une amine primaire ou secondaire choisie dans le groupe constitué par les amines aliphatiques, telles que la méthylamine, l'éthylamine, la propylamine, la butylamine, la pentylamine, l'hexylamine, l'heptylamine, l'octylamine, la dodécylamine, l'isopropylamine, l'isobutylamine et la benzylamine, ainsi que l'allylamine, les amines cycloaliphatiques primaires, telles que la cyclopropylamine, la cyclobutylamine, la cyclopentylamine et la cyclohexylamine, les amines aromatiques telles que l'aniline, les aminotoluènes isomères, individuellement ou en mélanges, et les xylidines isomères, individuellement ou en mélanges, qui peuvent éventuellement être substituées par un ou plusieurs halogènes, et les amines aliphatiques secondaires, la diméthylamine, la méthyléthylamine, la diéthylamine, la dipropylamine, la diisopropylamine, la dibutylamine, la dipentylamine, la dihexylamine, la diheptylamine et la dioctylamine.

3. Procédé de fabrication d'amides de l'acide (méth)acrylique selon les revendications 1 à 2, **caractérisé en ce que** la liqueur mère de l'étape de séparation de l'amide est neutralisée, afin de précipiter davantage d'amide, et l'amide formé est séparé par filtration.

4. Procédé de fabrication d'amides de l'acide (méth)acrylique selon les revendications 1 à 2, **caractérisé en ce que** la liqueur mère de l'étape de séparation de l'amide est débarrassée de l'acide (méth)acrylique par distillation, et l'amide restant est séparé par filtration.

5. Procédé de fabrication de N-isopropylméthacrylamide, **caractérisé en ce que** de l'anhydride de l'acide méthacrylique est mis en réaction avec de l'isopropylamine dans de l'eau, et le N-isopropylméthacrylamide est isolé.
